# EUROPEAN PATENT APPLICATION

(11) **EP 4 778 483 A1**
(43) Date of publication of application: **22.07.2026**
(21) Application number: 25152266.0
(22) Date of filing: 16.01.2025
(51) Int. Cl.: A61B 18/18

(54) **LIGHT-BASED SKIN TREATMENT DEVICE, METHOD OF REMOVING HAIRS, AND DEVICE MOVEMENT GUIDE**

(71) Applicant: Koninklijke Philips N.V., 5656 AE Eindhoven (NL)
(72) Inventor: ZHANG, Jun, Eindhoven (NL); LIN, Xiaoyu, Eindhoven (NL); JIANG, Ou, Eindhoven (NL)
(74) Representative: Philips Intellectual Property & Standards

(57) **Abstract**

There is provided a light-based skin treatment device, comprising a light source, a treatment window, and a device movement guide. The light source is adapted to generate treatment light. The treatment window is adapted to expose a first treatment area of a skin with treatment light when the device is in a first exposure position, and to expose a second treatment area of the skin with treatment light when the device is in a second exposure position. The device movement guide is adapted to guide a user to move the device along the skin in a movement direction from the first exposure position to the second exposure position. A distance between the first exposure position and the second exposure position in the movement direction is more than 100% and less than 120% of a width of the treatment window. The device movement guide is configured to generate a treatment signal when the device is in the first exposure position and when the device is in the second exposure position.

## Description

### FIELD OF THE INVENTION

The invention relates to a light-based skin treatment device, such as an intense pulsed light (IPL) device. The invention further relates to a method of removing hairs with a light-based skin treatment device. The invention further relates to a device movement guide for use in a light-based skin treatment device.

### BACKGROUND OF THE INVENTION

Light-based skin treatment devices are known to perform photo-epilation for hair removal and hair growth reduction. Home-use consumer devices are commercially available, such as the Lumea (TM) of Philips (TM). Some home-use devices use Intense Pulsed Light technology (IPL) using e.g. a Xenon flash lamp at a relatively low fluence (up to 6.5 J/cm²), as compared to professional devices for permanent photo-epilation, that use fluences in excess of 10 J/cm².

IPL technology uses a hand-held flash lamp to deliver an intense, visible, broad-spectrum pulse of treatment light. The treatment light is generally in the visible spectral range of 400 to 1200 nm. Cutoff filters are, for example, used to selectively filter out shorter wavelengths, especially potentially damaging ultra violet light. The resulting treatment light has a spectral range that targets specific structures and chromophores, in particular the melanin pigment in hair.

The light absorbed by melanin in the hair and hair matrix generates heat that puts the hair follicles in a sleep state. When the treatment is repeated in intervals of 2 to 4 weeks, a long-lasting hair reduction result is obtained.

To treat a complete body part, such as a whole leg, the device is moved by the user along the body part. The user holds the device at a position at which the device irradiates the skin with a pulse of treatment light. The user then moves the device to a next position at which the device again irradiates the skin with a pulse of treatment light. This process is repeated till the user has moved the device along the entire body part.

### SUMMARY OF THE INVENTION

The user is not able to see which part of the skin has already been irradiated and which part has not. As a result, many users struggle to hold the device at the correct positions to irradiate the entire body part in a uniform way. In case the user does not move the device over a sufficiently large distance between irradiations, parts of the skin are irradiated multiple times. Irradiating the skin multiple times may lead to discomfort, pain or even burns. In case the user moves the device over a too large distance between irradiations, parts of the skin are not irradiated. The hairs in these parts of the skin remain. This may result in poor depilation results.

In it an objective to improve guidance of the user when moving a light-based skin treatment device.

According to a first aspect, there is provided a light-based skin treatment device comprising a light source, a treatment window, and a device movement guide. The light source is adapted to generate treatment light. The treatment window is adapted to expose a first treatment area of a skin with treatment light when the device is in a first exposure position, and to expose a second treatment area of the skin with treatment light when the device is in a second exposure position. The device movement guide is adapted to guide a user to move the device along the skin in a movement direction from the first exposure position to the second exposure position. A distance between the first exposure position and the second exposure position in the movement direction is more than 100% and less than 120% of a width of the treatment window. The width of the treatment window is parallel to the movement direction. The device movement guide is configured to generate a treatment signal when the device is in the first exposure position and when the device is in the second exposure position, and generate no treatment signal when the device is in between the first exposure position and the second exposure position. The treatment signal is representative of the device being in a position to expose an area of the skin.

When the user has used the device to irradiate the first treatment area, the device movement guide helps the user to move in the movement direction towards the second exposure position. While the user is moving the device towards the second exposure position, no treatment signal is generated. As soon as the user has moved the device in the second exposure position, the treatment signal is generated. The treatment signal indicates to the user or to the device itself that the device is in a proper position to expose the skin to the treatment light. Because the distance between the first exposure position and the second exposure position in the movement direction is more than 100% of the width of the treatment window, the skin irradiated when the device is at the second exposure position was not irradiated with the treatment light when the device was at the first exposure position. Therefore, over-exposure of the skin is prevented or reduced. Because the distance is less than 120% of the width of the treatment window, only a small part of the skin is not irradiated. The few hairs in that part of the skin do not substantially affect the outcome of the treatment. Because this part of the skin is small, and because hairs are spaced apart, the impact on hair removal efficiency is minimal. As a result, the improved guidance of the user when moving a light-based skin treatment device maintains a good hair removal efficiency while reducing the risk of overtreatment and burns.

The light source is adapted to provide the treatment light, for example in light pulses. For example, the light source is adapted to generate light at a high intensity for a short duration, such as for less than 100 ms or less than 50 ms or less than 10 ms or less than 8 ms. The intensity of the light pulse is high enough to perform the treatment on the skin. Such treatment is, for example, hair removal or photo-rejuvenation or vein treatment or acne treatment. For example, the light source comprises one or more optical filters. For example, the light source comprises an optical filter to prevent light with a potentially damaging wavelength, such as UV light, from being transmitted to the skin. For example, the light source comprises a flash lamp such as a Xenon flash lamp, or a laser, or multiple lasers, or a LED, or multiple LEDs. For example, the light source is adapted to provide the treatment light with wavelengths in the range of 530-1200 nanometers. For example, the light-based skin treatment device is an intense pulsed light (IPL) device. For example, the light-based skin treatment device is an intense pulsed light (IPL) device adapted for photo-epilation.

The treatment window is adapted to expose a part of the skin with treatment light from the light source. The treatment window comprises, for example, an optical transparent material that is transparent to the treatment light. For example, the treatment window has a rectangular shape, such as a rectangular shape with rounded corners. For example, the width of the treatment window is the width of the rectangular shape in the movement direction. For example, the treatment window has a round shape. For example, the width of the treatment window is the diameter of the round shape. For example, the treatment window has a square shape. For example, the width of the treatment window is the edge of the square shape.

The first treatment area is a part of the skin that is exposed to treatment light when the device is in the first exposure position. For example, the first treatment area has the same size and shape as the treatment window. For example, the first treatment area has a larger size than the treatment window. For example, the first treatment area has the larger size, because of a distance between the skin and the treatment window. The treatment light exiting the treatment window may diverge before reaching the skin, causing the first treatment area to have a larger size than the treatment window.

The second treatment area is a part of the skin that is exposed to treatment light when the device is in the second exposure position. For example, the size and/or shape of the second treatment area is the same as that of the first treatment area. The size and/or shape of the second treatment area may be different than the first treatment area, for example, caused by the geometry of the body part that is being treated. When the device is moved from the first exposure position to the second exposure position as guided by the device movement guide, the first treatment area and the second treatment area are adjacent to each other with a small untreated skin area in between. The untreated skin area is not exposed to the treatment light.

The device movement guide is adapted to guide the user to move the device along the skin in a movement direction from the first exposure position to the second exposure position. For example, the device movement guide comprises a visual mark, such as an arrow, to indicate the movement direction. For example, the device movement guide comprises a guide light source adapted to project a light spot or a light pattern on the skin. The light spot or the light pattern indicates the movement direction and/or where the device will be at the second exposure position. The user is able to move the device towards the light spot or light pattern to move the device towards the second exposure position.

The device movement guide is configured to generate the treatment signal. The treatment signal is, for example, a perceivable signal that is perceivable for the user. For example, the perceivable signal is a visual signal, or an auditory signal, or a tactile signal. A tactile signal comprises, for example, a vibration signal. When the user perceives the perceivable signal, the user is made aware by the device movement guide that the device is at the second exposure position. The user directly understands to operate the device to irradiate the skin with the treatment light. For example, the device has a button that the user can press to operate the device to expose the skin in response to the perceivable signal. For example, the device movement guide comprises an indicator light that generates a green light in response to the treatment signal. For example, the indicator light does not generate the green light in absence of the treatment signal.

The device movement guide is configured not to generate the treatment signal when the device is in between the first exposure position and the second exposure position. The absence of the treatment signal is representative of the device being away from the second exposure position. For example, in case of the treatment signal being the perceivable signal, the user is not provided with any perceivable signal while moving the device towards the second exposure position. Therefore, the user is not prompted to operate the device to irradiate the skin. The user will therefore not press any button to operate the device to irradiate the skin until the perceivable signal is perceived. For example, the device movement guide is configured to generate a non-treatment signal when the device is in between the first exposure position and the second exposure position. For example, the non-treatment signal is a perceivable signal that is perceivable to the user. For example, the device movement guide comprises an indicator light that generates a red light in response to the non-treatment signal. For example, the indicator light does not generate the red light in response to the treatment signal.

In an embodiment, wherein the distance is in the range of 105%-110% of the width of the treatment window.

According to this embodiment, the distance of 105% or more of the width of the treatment window prevents overlap between the first treatment area and the second treatment area, even when taking uncertainties into account, such as errors in determining the position of the device, and the geometry of the body part. The distance of 110% or less of the width of the treatment window results in such a small area of skin between the first treatment area and the second treatment area, that the remaining hairs in that small area of skin have no or almost no negative effect on the treatment outcome.

In an embodiment, wherein the treatment signal comprises a tactile signal.

According to this embodiment, the device movement guide generates a tactile signal when the device is in the first exposure position and in the second exposure position. As the user holds the device while moving the device from the first exposure position towards the second exposure position, the user can feel the tactile signal when the device arrives at the second exposure position. The tactile signal directs the user to operate the device to irradiate the skin. For example, the device movement guide is adapted to provide a mechanical shock, or clap, or thump to the device as the tactile signal. For example, the device movement guide comprises a vibrator adapted to generate a vibration in response to the treatment signal. The vibration is the tactile signal.

In an embodiment, the device movement guide comprises a roller arranged to contact the skin. The roller is adapted to rotate along an axis perpendicular to the movement direction when moved along the skin in the movement direction. The roller has a polygon shape having sides. Each of the sides corresponds to the distance.

According to this embodiment, as the device is moved along the skin from the first exposure position towards the second exposure position, the roller rotates along the axis. While moving the device, the user can feel when the roller has rolled from one side in contact with the skin to a next side in contact with the skin. The user can feel this because of the unround, polygon shape of the roller. As the sides corresponds to the distance, the user has moved the device over the distance between the first exposure position to the second exposure position, when the user feels that the roller has rolled from one side in contact with the skin to the next side in contact with the skin. This way, the roller indicates to the user when the device is at the second exposure position, at which the user can operate the device to irradiate the second treatment area.

In an embodiment, the roller comprises a plurality of markings arranged on the sides. The markings are visible to the user. The markings form at least part of the treatment signal.

According to this embodiment, the markings are arranged on a lateral surface of the roller and move along with the rotation of the roller. The markings and the roller are arranged to allow the user to see the markings. Based on the markings, the user is able to see whether the device is in the second exposure position or not. For example, the marking is not visible or not clearly visible when the device is in between the first exposure position and the second exposure position. For example, the marking is aligned with a stationary marking arranged on the device. The device is at the second exposure position when the marking on the roller is aligned with the stationary marking, and the device is in between the first exposure position and the second exposure position when the marking is not aligned with the stationary marking. For example, the markings are different for different sides of the polygon shape. Because the markings are different for different sides of the polygon shape, the user is better able to perceive the rotation of the roller. This way, it is less likely the user accidentally moves the device a distance corresponding to multiple sides of the roller in a single movement without irradiating the skin. For example, the markings comprise multiple numbers, such as the numbers 1-6 for a hexagonal shape of the roller. For example, the markings comprise an alternating coloring of the sides, such as alternating black and white.

In an embodiment, the light-based skin treatment device comprises a main housing holding the light source. The roller is adapted to be attachable and detachable from the main housing.

According to this embodiment, the roller is attachable to the main housing to guide the user. Since the roller increases the size of the device, the roller is, for example, attached when the user treats large body parts, such as the legs. However, when the user wants to treat a small body part, such as an upper lip, the roller may be too large to be used. In that situation, the user is able to detach the roller from the main housing. The user is able to use the device without the roller for the small body part. Alternatively, the user attaches a smaller roller to the main housing for treating the small body part.

In an embodiment, the light-based skin treatment device comprises a controller adapted to control the light source to generate treatment light in response to the treatment signal.

According to this embodiment, the controller is able to receive the treatment signal. In response to receiving the treatment signal, the control controls the light source to generate the treatment light. Without receiving the treatment signal, the controller controls the light source not to generate treatment light. As a result, the treatment light is generated only in response to the treatment signal. This allows the treatment light to irradiate the first treatment area when the device is in the first exposure position and the second treatment area when the device is in the second exposure position. It prevents the skin from being irradiated with treatment light when the device is in between the first exposure position and the second exposure position. As the controller controls the operation of the light source, the user is able to move the device along the body part without the need for the user to operate the device to generate the treatment light.

In an embodiment, the light-based skin treatment device comprises a controller adapted to block the light source from generating treatment light in absence of the treatment signal.

According to this embodiment, the controller blocks the light source from generating treatment light if there is no treatment signal. This prevents the generation of treatment light when the device is in between the first exposure position and the second exposure position. If there is no treatment signal, no treatment light is generated, for example, even if the user operates the button to operate the device to expose the skin. The controller is adapted not to block the light source from generating treatment light in response to the treatment signal.

In an embodiment, the light-based skin treatment device comprises an acoustic generator adapted to generate an acoustic signal in response to the treatment signal.

According to this embodiment, the acoustic generator generates the acoustic signal in response to the treatment signal, thereby indicating that the device is at the second exposure position. For example, the acoustic signal is a beep or a ping or a voice message. The voice message, for example, indicates the user to flash now. For example, the acoustic generator is adapted to generate another acoustic signal in absence of the treatment signal. This acoustic signal is different from the other acoustic signal to clearly communicate to the user when the device is at the second exposure position.

In an embodiment, the device movement guide comprises a motion sensor. The motion sensor is adapted to generate a motion signal representative of motion of the light-based skin treatment device. The device movement guide is adapted to guide the user based on the motion signal.

According to this embodiment, the device movement guide is able to determine the movement of the device since the exposure of the first treatment area. By determining the movement, the device movement guide is able to determine whether the device has moved the distance between the first exposure position and the second exposure position. When the device movement guide determines, based on the motion signal, that the device is at the second exposure position, the device movement guide generates the treatment signal.

In an embodiment, the motion sensor comprises at least one of an optical displacement sensor or an accelerometer.

According to this embodiment, the device movement guide uses the optical displacement sensor and/or the accelerometer to determine whether the device has moved the distance between the first exposure position and the second exposure position. The optical displacement sensor and/or the accelerometer are cost-effective sensors that provide sufficient accuracy to determine the movement of the device.

In a second aspect of the invention, there is provided a method of removing hairs with a light-based skin treatment device. The method comprises exposing a first treatment area of a skin with the light-based skin treatment device at a first exposure position. The method comprises guiding a user to move the device along the skin in a movement direction over a distance from the first exposure position to a second exposure position. The distance is more than 100% and less than 120% of a width of the first treatment area. The width of the first treatment area is parallel to the movement direction. The method comprises generating no treatment signal when the device is between the first exposure position and the second exposure position. The method comprises generating a treatment signal when the device is in the second exposure position. The treatment signal is representative of the device being in a position to expose an area of the skin. The method comprises exposing a second treatment area of the skin with the light-based skin treatment device at the second exposure position.

When the first treatment area is exposed, the user is guided to move the device along the skin in the movement direction towards the second exposure position. While the user is moving the device towards the second exposure position, no treatment signal is generated. As soon as the user has moved the device to the second exposure position, the treatment signal is generated. The treatment signal indicates to the user or to the device itself that the device is in a proper position to expose the skin with the treatment light. Because the distance between the first exposure position and the second exposure position in the movement direction is more than 100% of the width of the first treatment area, the skin irradiated when the device is at the second exposure position was not irradiated with the treatment light when the device was at the first exposure position. Therefore, over-exposure of the skin is prevented or reduced. Because the distance is less than 120% of the width of the first treatment area, only a small part of the skin is not irradiated. The few hairs in that part of the skin do not substantially affect the outcome of the treatment. Because this part of the skin is small, and because hairs are spaced apart, the impact on hair removal efficiency is minimal. As a result, the improved guidance of the user when moving a light-based skin treatment device maintains a good hair removal efficiency while reducing the risk of overtreatment and burns.

For example, the method is a non-therapeutic method of removing hairs with a light-based skin treatment device. For example, the method is a cosmetic method of removing hairs with a light-based skin treatment device. For example, the method is a non-therapeutic, cosmetic method of removing hairs with a light-based skin treatment device.

In an embodiment, the distance is in the range of 105%-110% of the width of the first treatment area.

According to this embodiment, the distance of 105% or more the width of the first treatment area prevents overlap between the first treatment area and the second treatment area, even when taking uncertainties into account, such as errors in determining the position of the device and the geometry of the body part. The distance of 110% or less the width of the first treatment area results in such a small area of skin between the first treatment area and the second treatment area, that the remaining hairs in that small area of skin have no or almost no negative effect on the treatment outcome.

In an embodiment, guiding the user comprises moving a roller along the skin and rolling the roller along an axis perpendicular to the movement direction. The roller has a polygon shape having sides. Each of the sides correspond to the distance.

In a third aspect of the invention there is provided a device movement guide comprising a roller adapted to be used in the light-based skin treatment device according to the first aspect.

### BRIEF DESCRIPTION OF THE DRAWINGS

Exemplary embodiments will now be described, by way of example only, with reference to the following figures, in which:
FIG. 1 depicts a light-based skin treatment device according to a first embodiment of the invention;
FIG. 2 depicts a front view of the light-based skin treatment device according to the first embodiment;
FIG. 3 depicts the light-based skin treatment device of the first embodiment in a first exposure position and in a second exposure position;
FIGs. 4-6 depict the light-based skin treatment device according to a second embodiment;
FIG. 7 depicts the roller according to a third embodiment;
FIG. 8 depicts the light-based skin treatment device according to a fourth embodiment;
FIG. 9 depicts a device movement guide according to a fifth embodiment;
FIG. 10 depicts a method of removing hairs with a light-based skin treatment device;
FIG. 11 depicts a top view of a first treatment area and a second treatment area.

### DETAILED DESCRIPTION OF EMBODIMENTS

It should be understood that the detailed description and specific examples, while indicating exemplary embodiments of the device and method, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the figures are merely schematic and are not drawn to scale. It should also be understood that the same reference numerals are used throughout the figures to indicate the same or similar parts.

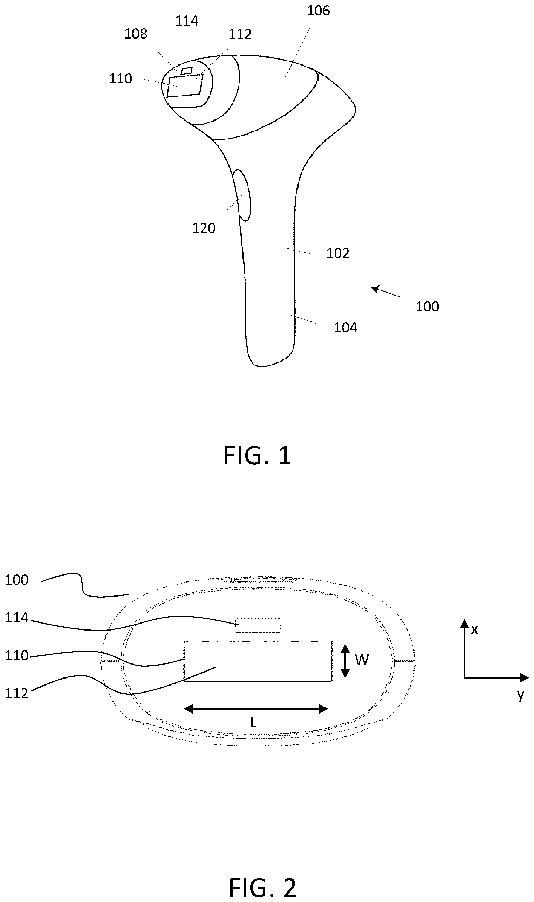

FIG. 1 depicts a light-based skin treatment device, in this case an Intense Pulsed Light, IPL, device 100 according to a first embodiment of the invention. The IPL device 100 is adapted for use on the skin of a subject, e.g., a person or an animal. The IPL apparatus 100 comprises a housing 102 that has a handle portion 104 and a head portion 106. The handle portion 104 is shaped to enable the user to hold the IPL device 100. The head portion 106 has a head end 108 that is to be placed into contact with the subject in order for the IPL apparatus 100 to perform a treatment operation on the skin of the subject.

The IPL device 100 comprises a light source adapted to generate treatment light. The light source is arranged inside the head portion 106. The IPL device 100 performs the treatment operation by providing pulses of the treatment light to the skin via the aperture 110. The aperture 110 is arranged in or on the housing 102 so that the aperture 110 can be placed adjacent to or on (i.e. in contact with) the skin of the subject. A treatment window 112 is arranged in the aperture 110. The intensity of the light pulses from the light source are high enough to perform the treatment operation on the skin or body part adjacent to the aperture 110. For example, the treatment operation is photo-epilation.

The IPL device 100 comprises a user control 120 adapted to be operated by the user to activate the IPL device 100. When the user activates the IPL device 100 via the user control 120, the IPL device 100 is able to perform the treatment operation. The user control 120 may be in the form of a switch, a button, a touch pad, etc.

In a setting at home, the user handling the IPL device 100 places the head end 108 in contact with the user's own skin. In a commercial setting, such as a beauty salon or a spa, a professional user may handle the IPL device 100 and may place the head end 108 in contact with the skin of another person, e.g., a client of the beauty salon.

A sensor window 114 is provided. A sensor is arranged in the head end 108 to provide an optical signal to the skin via the sensor window 114, and to receive a reflected optical signal from the skin via the sensor window 114. The sensor is adapted to generate a skin tone signal based on the reflected optical signal. The skin tone signal is representative of a skin tone of the skin facing the sensor window 114. In an embodiment, the sensor and the sensor window 114 are omitted.

FIG. 2 depicts a front view of the IPL device 100 according to the first embodiment. The front view depicts the aperture 110, the treatment window 112 and the sensor window 114.

To explain the embodiments of the invention, use is made of a cartesian coordinate system having an x-axis, a y-axis, and a z-axis. As shown in FIG. 2, the treatment window 112 has a rectangular shape. The size of the rectangular shape along the x-axis is referred to as the width W of the treatment window 112. The size of the rectangular shape along the y-axis is referred to as the length L of the treatment window 112.

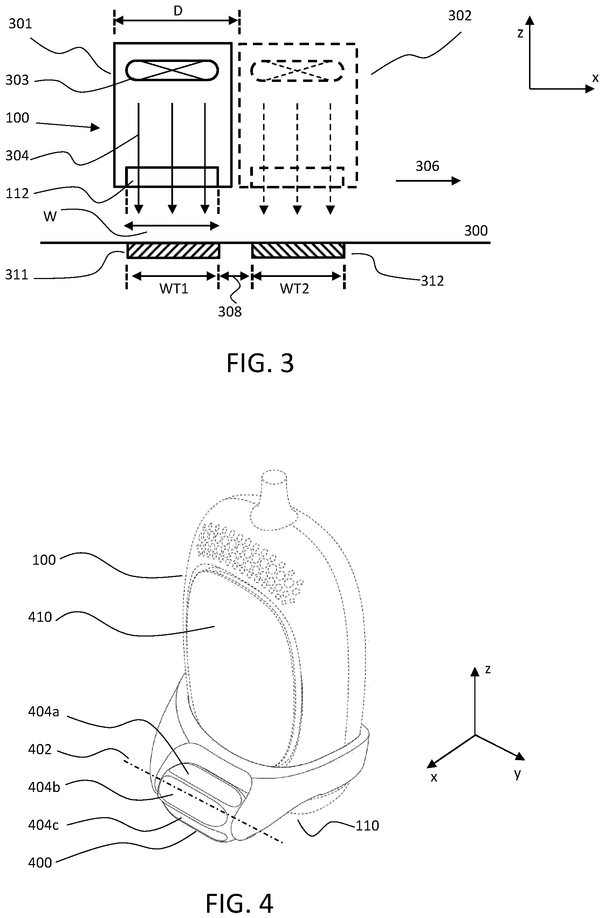

FIG. 3 depicts the IPL device 100 of the first embodiment in a first exposure position 301 and in a second exposure position 302. The IPL device 100 in the first exposure position 301 is indicated with solid lines, whereas the IPL device 100 in the second exposure position 302 is indicated with dashed lines. The IPL device 100 faces the skin 300. The treatment window 112 faces the skin 300. As indicated by the coordinate system, the z-axis is perpendicular to the skin 300. The x-axis is parallel to the skin 300. The IPL device 100 comprises the light source 303 adapted to generate the treatment light 304. When the IPL device 100 is in the first exposure position 301, the treatment window 112 is adapted to expose the first treatment area 311 of the skin 300 with treatment light 304 from the light source 303.

Then, the IPL device 100 is moved in a movement direction 306 along the x-axis from the first exposure position 301 to the second exposure position 302. When the IPL device 100 is in the second exposure position 302, the treatment window 112 is adapted to expose the second treatment area 312 of the skin 300 with treatment light 304. For example, the treatment light 304 is provided as a pulse of light.

A device movement guide, which will be illustrated in following figures, is adapted to guide the user to move the IPL device 100 along the skin 300 in the movement direction 306 from the first exposure position 301 to the second exposure position 302. The distance D between the first exposure position 301 and the second exposure position 302 in the movement direction 306 is more than 100% and less than 120% of the width W of the treatment window 112. The width W of the treatment window 112 is parallel to the movement direction 306.

Because of the size of the distance D, the first treatment area 311 and the second treatment area 312 have a gap 308 in between. The gap 308 is formed by skin 300 that was not exposed to treatment light 304 when the IPL device 100 was in the first exposure position 301 or when the IPL device 100 was in the second exposure position 302. For example, the distance D is in the range of 105%-110% of the width W of the treatment window 112.

The first treatment area 311 has a width WT1. The second treatment area 312 has a width WT2.

FIGs. 4-6 depict the IPL device 100 according to a second embodiment. The second embodiment has, for example, the same features as the first embodiment, except for the following. In the second embodiment, the device movement guide generates a tactile signal. The tactile signal forms a treatment signal. The device movement guide generates the treatment signal when the IPL device 100 is in the first exposure position 301 and when the IPL device 100 is in the second exposure position 302. The device movement guide generates no treatment signal when the IPL device 100 is in between the first exposure position 301 and the second exposure position 302. The treatment signal is representative of the IPL device 100 being in a position to expose an area of the skin 300. When the user feels the tactile signal, the user is prompted to operate the user control 120 to provide treatment light 304 to the skin 300.

In the second embodiment, the device movement guide comprises a roller 400 arranged to contact the skin 300. The roller 400 is adapted to rotate along an axis 402 perpendicular to the movement direction 306 when moved along the skin 300 in the movement direction 306. The roller 400 has a polygon shape having sides 404a-404d. Each of the sides 404a-404d corresponds to the distance D.

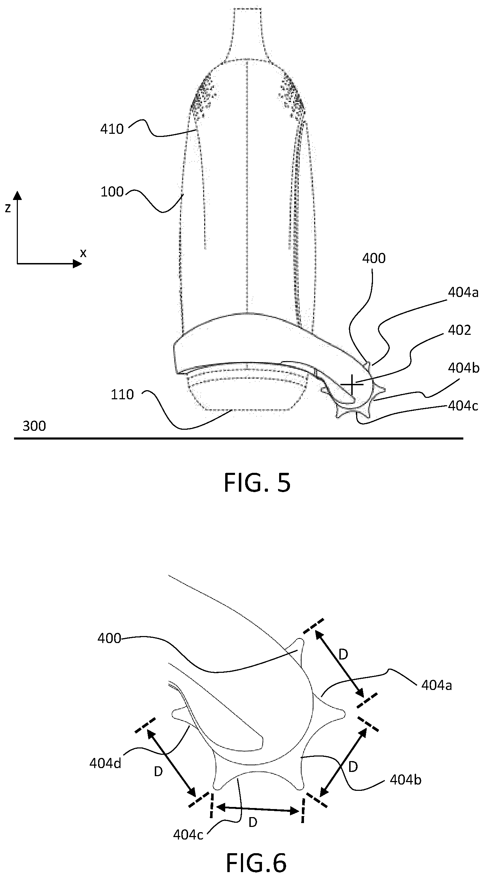

FIG. 5 depicts a side view of the second embodiment while in operational use. The treatment window 112 faces the skin 300 to be able to expose the skin 300. The figure shows a distance between the IPL device 100 and the skin 300 to show the elements of the IPL device 100 more clearly. In practice, the IPL device 100 may be in contact with the skin 300. For example, the roller 400 may cause a small indentation of the skin 300. The figure shows that the roller 400 has a star-shape, which is an example of a polygon shape. When moving the IPL device 100 along the x-axis, the roller 400 will rotate along the axis 402. The polygon shape will cause the user to feel when the roller 400 brings the next side of sides 404a-404d into contact with the skin 300. The polygon shape causes a gentle shaking of the IPL device 100 while rolling along the skin 300. This way, the user is made aware when the IPL device 100 is in the correct position to expose the skin 300 to the treatment light 304.

FIG. 6 depicts a close up view of the roller 400. Each of the sides 404a-404d has a length equal to the distance D. As a result, every time the roller 400 rolls from one side of sides 404a-404d to the adjacent side, the IPL device 100 is moved over a distance D.

For example, the roller 400 comprises a plurality of markings arranged on the sides 404a-404d. The markings are visible to the user. The roller 400 has at least some sides of sides 404a-404d that face away from the skin 300 at any time. The markings form at least part of the treatment signal.

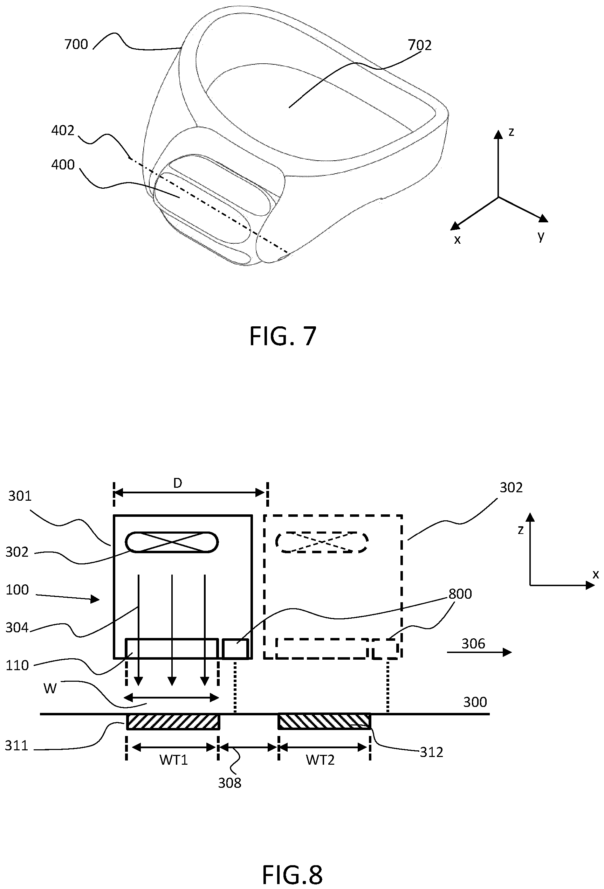

The IPL device 100 has a main housing 410. The main housing 410 holds the light source 303. In a third embodiment, the roller 400 is adapted to be attachable and detachable from the main housing 410, as is depicted in FIG. 7.

FIG. 7 depicts the roller 400 according to the third embodiment. The third embodiment has, for example, the same features as the first embodiment, or the second embodiment, except for the following. The roller 400 is rotationally connected to a frame 700. The roller 400 is rotatably connected to the frame 700 to rotate along the axis 402 relative to the frame 700. The frame 700 has an opening 702 to receive the main housing 410 of the IPL device 100. For example, the main housing 410 is clamped into the opening 702 to fixate the frame 700 to the main housing 410.

FIG. 8 depicts the IPL device 100 according to a fourth embodiment. The fourth embodiment has, for example, the same features as the first embodiment, the second embodiment, or the third embodiment, except for the following.

In the fourth embodiment, the device movement guide comprises a motion sensor 800. The motion sensor 800 is adapted to generate a motion signal representative of motion of the light-based skin treatment device 100. The device movement guide is adapted to guide the user based on the motion signal.

The motion sensor 800 is an optical displacement sensor. The optical displacement sensor provides an optical signal to the skin 300. Based on the reflection of the optical signal by the skin 300, the optical displacement sensor generates the motion signal. For example, the optical displacement sensor is similar to an optical mouse tracker. As alternative or in addition, the motion sensor 800 comprises an accelerometer, such as an inertial measurement unit, IMU.

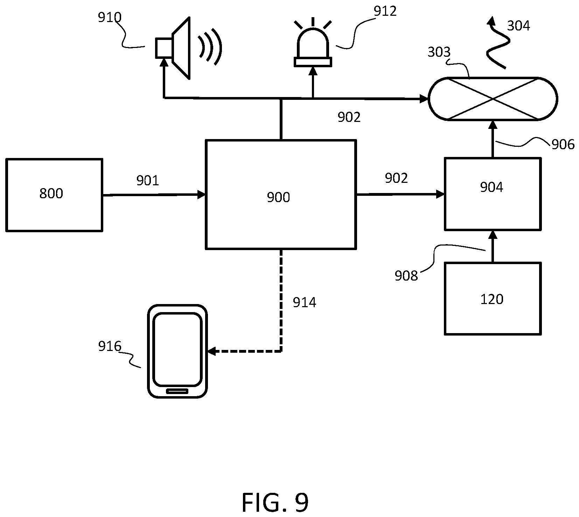

FIG. 9 depicts the device movement guide 900 of IPL device 100 according to a fifth embodiment. The fifth embodiment has, for example, the same features as the first embodiment, the second embodiment, the third embodiment, or the fourth embodiment except for the following. The device movement guide 900 of the fifth embodiment is, for example, used in the IPL device 100 any one of the first embodiment, the second embodiment, the third embodiment, or the fourth embodiment.

The device movement guide 900 is configured to receive the motion signal 901 from the motion sensor 800. The device movement guide 900 is adapted to determine a position or a displacement of the IPL device based on the motion signal 901.

The device movement guide 900 is configured to generate a treatment signal 902 when the IPL device 100 is in the first exposure position 301 and when the IPL device 100 is in the second exposure position 302. The device movement guide 900 is configured to generate no treatment signal 902 when the IPL device 100 is in between the first exposure position 301 and the second exposure position 302. The treatment signal 902 is representative of the IPL device 100 being in a position to expose an area of the skin 300.

For example, the IPL device 100 comprises a controller 904 adapted to control the light source 303 to generate treatment light 304 in response to the treatment signal 902. The device movement guide 900 provides the treatment signal 902 to the controller 904. The controller 904 provides a control signal 906 to the light source 303. The control signal 906 causes the light source 303 to generate treatment light 304.

For example, the controller 904 adapted to block the light source 303 from generating treatment light 304 in absence of the treatment signal 902. When the user operates the user control 120, the user control 120 provides a user signal 908 to the controller 904. The controller 904 is configured to generate the control signal 906 for the light source 303 only in case the controller 904 receives the user signal 908 and the treatment signal 902. In absence of the treatment signal 902, the controller 904 does not provide the control signal 906 to the light source 303, despite receiving the user signal 908.

For example, the IPL device 100 comprises an acoustic generator 910 adapted to generate an acoustic signal in response to the treatment signal 902. The acoustic generator 910 is, for example, a loudspeaker. When the device movement guide 900 generates the treatment signal 902, the acoustic generator 910 provides an acoustic signal, such as a beep or sound or voice message. The acoustic signal prompts the user to operate the user control 120. The controller 904 receives the user signal 908 from the user control 120 and provides the control signal 906 to the light source 303. The light source 303 provides treatment light 304 in response to the control signal 906. Optionally, the device movement guide 900 provides the treatment signal 902 to both the acoustic generator 910 and the controller 904. The controller 904 is configured to generate the control signal 906 for the light source 303 only in case the controller 904 receives the user signal 908 and the treatment signal 902. In absence of the treatment signal 902, the controller 904 does not provide the control signal 906 to the light source 303.

For example, the IPL device 100 comprises a visual signal generator 912 adapted to generate a visual signal in response to the treatment signal 902. For example, the visual signal generator 912 comprises an LED or an array of LEDs. When the device movement guide 900 generates the treatment signal 902, the visual signal generator 912 provides a visual signal, such as a green light, or a flash, or a visual pattern. The visual signal prompts the user to operate the user control 120. The controller 904 receives the user signal 908 from the user control 120 and provides the control signal 906 to the light source 303. The light source 303 provides treatment light 304 in response to the control signal 906. Optionally, the device movement guide 900 provides the treatment signal 902 to both the visual signal generator 912 and the controller 904. The controller 904 is configured to generate the control signal 906 for the light source 303 only in case the controller 904 receives the user signal 908 and the treatment signal 902. In absence of the treatment signal 902, the controller 904 does not provide the control signal 906 to the light source 303.

For example, the device movement guide 900 is adapted to generate a display signal 914. The device movement guide 900 is adapted to provide the display signal 914 to a display or a device having display, such as a mobile phone 916. When the device movement guide 900 generates the treatment signal 902, the device movement guide 900 generates the display signal 914. The display signal 914 causes the display to show an image or text on the display to the user to operate the user control 120. The controller 904 receives the user signal 908 from the user control 120 and provides the control signal 906 to the light source 303. The light source 303 provides treatment light 304 in response to the control signal 906. Optionally, the device movement guide 900 provides the treatment signal 902 to both the visual signal generator 912 and the controller 904. The controller 904 is configured to generate the control signal 906 for the light source 303 only in case the controller 904 receives the user signal 908 and the treatment signal 902. In absence of the treatment signal 902, the controller 904 does not provide the control signal 906 to the light source 303.

In a further embodiment, there is provided a device movement guide 900 comprising the roller 400 adapted to be used in the IPL device 100 according to any one of the first embodiment, the second embodiment, the third embodiment, or the fourth embodiment.

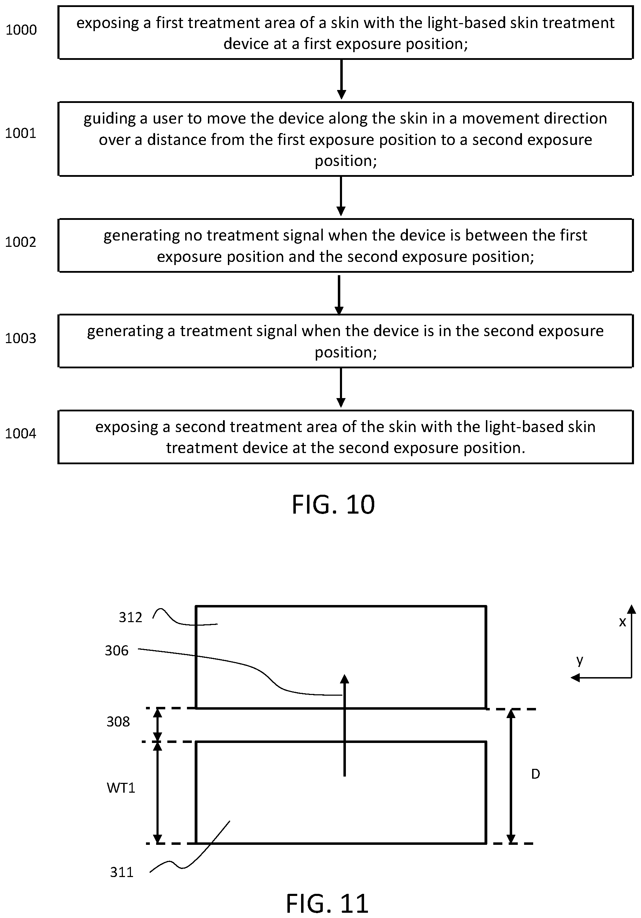

FIG. 10 depicts a method of removing hairs with a light-based skin treatment device, such as IPL device 100. The method may be performed with an IPL device 100 according to any one of the first embodiment, the second embodiment, the third embodiment, or the fourth embodiment.

The method comprises, at 1000, exposing a first treatment area 311 of a skin 300 with the light-based skin treatment device 100 at a first exposure position 301. The method comprises, at 1001, guiding a user to move the light-based skin treatment device 100 along the skin 300 in a movement direction 306 over a distance D from the first exposure position 301 to a second exposure position 302. The distance D is more than 100% and less than 120% of the width WT1 of the first treatment area 311. The width WT1 of the first treatment area 311 is parallel to the movement direction 306. The method comprises, at 1002, generating no treatment signal 902 when the light-based skin treatment device 100 is between the first exposure position 301 and the second exposure position 302. The method comprises at 1003, generating a treatment signal 902 when the light-based skin treatment device 100 is in the second exposure position 302. The treatment signal 902 is representative of the light-based skin treatment device 100 being in a position to expose an area of the skin 300. The method, comprises at 1004, exposing a second treatment area 312 of the skin 300 with the light-based skin treatment device 100 at the second exposure position 302.

For example, the distance D is in the range of 105%-110% of the width of the first treatment area 311.

For example, guiding the user comprises moving the roller 400 along the skin 300 and rolling the roller 400 along an axis 402 perpendicular to the movement direction 306. The roller 400 has a polygon shape having sides. Each of the sides correspond to the distance D.

FIG. 11 depicts a top view of the first treatment area 311 and the second treatment area 312. The first treatment area 311 is a portion of the skin 300 that is exposed to treatment light 304 when the IPL device 100 is in the first exposure position 301. After exposing the first treatment area 311, the IPL device 100 is moved in the movement direction 306 along the x-axis. The device movement guide 900 generates the treatment signal 902 when the IPL device 100 has reached the second exposure position 302. When the IPL device 100 is at the second exposure position 302, the second treatment area 312 is exposed to treatment light 304. The second treatment area 312 is a portion of the skin 300 that is exposed to treatment light 304 when the IPL device 100 is at the second exposure position 302.

The distance D is more than 100% and less than 120% of the width WT1 of the first treatment area 311. The width WT1 of the first treatment area 311 is parallel to the movement direction 306. For example, the distance D is in the range of 105%-110% of the width WT of the first treatment area 311. Because the distance D is more than 100% and less than 120% of the width WT of the first treatment area 311, the gap 308 of unexposed skin 300 is present between the first treatment area 311 and the second treatment area 312.

In an embodiment, the width W of the treatment window 112 is equal to the width WT1 of the first treatment area 311. In another embodiment, the width W of the treatment window 112 is different than the width WT1 of the first treatment area 311.

The device movement guide 900 and/or the controller 904 may each comprise a processor. The is adapted to perform the data processing. The processor can be implemented in numerous ways, with software and/or hardware, to perform the various functions required. The processor may employ one or more microprocessors that may be programmed using software (e.g., microcode) to perform the required functions. The processor may be implemented as a combination of dedicated hardware to perform some functions and one or more programmed microprocessors and associated circuitry to perform other functions.

The processor may include circuitry. Examples of circuitry that may be employed include, but are not limited to, conventional microprocessors, application specific integrated circuits (ASICs), and field-programmable gate arrays (FPGAs). Thus, the processor may be embodied as a digital and/or analog processing system.

In various implementations, the processor may be associated with one or more storage media such as volatile and non-volatile computer memory such as RAM, PROM, EPROM, and EEPROM. The storage media may be encoded with one or more programs that, when executed on one or more processing systems and/or controllers, perform the required functions. Various storage media may be fixed within a processor or may be transportable, such that the one or more programs stored thereon can be loaded into a processing system.

Functions implemented by a processor may be implemented by a single processor or by multiple separate processing units which may together be considered to constitute a "processor". Such processing units may in some cases be remote from each other and communicate with each other in a wired or wireless manner.

A computer program may be stored/distributed on a suitable medium, such as an optical storage medium or a solid-state medium supplied together with or as part of other hardware, but may also be distributed in other forms, such as via the Internet or other wired or wireless telecommunication systems. The processor may be configured to execute the computer program, causing the IPL device 100 to perform the method according to the sixth embodiment.

Variations to the disclosed embodiments can be understood and effected by those skilled in the art in practicing the principles and techniques described herein, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A light-based skin treatment device (100), comprising:
a light source (303) adapted to generate treatment light (304);
a treatment window (112) adapted to expose a first treatment area (311) of a skin (300) with treatment light (304) when the device is in a first exposure position (301), and to expose a second treatment area (312) of the skin (300) with treatment light (304) when the device is in a second exposure position (302); and
a device movement guide (900) adapted to guide a user to move the device along the skin (300) in a movement direction (306) from the first exposure position (301) to the second exposure position (302),
wherein a distance (D) between the first exposure position (301) and the second exposure position (302) in the movement direction (306) is more than 100% and less than 120% of a width of the treatment window (112),
wherein the width of the treatment window (112) is parallel to the movement direction (306);
wherein the device movement guide (900) is configured to:
generate a treatment signal (902) when the device is in the first exposure position (301) and when the device is in the second exposure position (302), and
generate no treatment signal (902) when the device is in between the first exposure position (301) and the second exposure position (302),
wherein the treatment signal (902) is representative of the device being in a position to expose an area of the skin (300).

2. The light-based skin treatment device (100) according to claim 1, wherein the distance (D) is in the range of 105%-110% of the width of the treatment window (112).

3. The light-based skin treatment device (100) according to any one of the preceding claims, wherein the treatment signal (902) comprises a tactile signal.

4. The light-based skin treatment device (100) according to claim 3, wherein the device movement guide (900) comprises a roller (400) arranged to contact the skin (300),
wherein the roller (400) is adapted to rotate along an axis (402) perpendicular to the movement direction (306) when moved along the skin (300) in the movement direction (306),
wherein the roller (400) has a polygon shape having sides (404a-d),
wherein each of the sides corresponds to the distance (D).

5. The light-based skin treatment device (100) according to claim 4, wherein the roller (400) comprises a plurality of markings arranged on the sides (404a-d),
wherein the markings are visible to the user,
wherein the markings form at least part of the treatment signal (902).

6. The light-based skin treatment device (100) according to claim 4 or 5, comprising a main housing (410) holding the light source (303),
wherein the roller (400) is adapted to be attachable and detachable from the main housing (410).

7. The light-based skin treatment device (100) according to any one of the preceding claims, comprising a controller (904) adapted to control the light source (303) to generate treatment light (304) in response to the treatment signal (902).

8. The light-based skin treatment device (100) according to any one of the preceding claims, comprising a controller (904) adapted to block the light source (303) from generating treatment light (304) in absence of the treatment signal (902).

9. The light-based skin treatment device (100) according to any one of the preceding claims, comprising an acoustic generator (910) adapted to generate an acoustic signal in response to the treatment signal (902).

10. The light-based skin treatment device (100) according to any one of the preceding claims,
wherein the device movement guide (900) comprises a motion sensor (800),
wherein the motion sensor (800) is adapted to generate a motion signal (901) representative of motion of the light-based skin treatment device (100),
wherein the device movement guide (900) is adapted to guide the user based on the motion signal (901).

11. The light-based skin treatment device (100) according to claim 10, wherein the motion sensor (800) comprises at least one of an optical displacement sensor or an accelerometer.

12. A method of removing hairs with a light-based skin treatment device (100), the method comprising:
exposing (1000) a first treatment area (311) of a skin (300) with the light-based skin treatment device (100) at a first exposure position (301);
guiding (1001) a user to move the device along the skin (300) in a movement direction (306) over a distance D from the first exposure position (301) to a second exposure position (302),
wherein the distance (D) is more than 100% and less than 120% of a width of the first treatment area (311),
wherein the width of the first treatment area (311) is parallel to the movement direction (306);
generating (1002) no treatment signal (902) when the device is between the first exposure position (301) and the second exposure position (302);
generating (1003) a treatment signal (902) when the device is in the second exposure position (302),
wherein the treatment signal (902) is representative of the device being in a position to expose an area of the skin (300);
exposing (1004) a second treatment area (312) of the skin (300) with the light-based skin treatment device (100) at the second exposure position (302).

13. The method according to claim 12, wherein the distance (D) is in the range of 105%-110% of the width of the first treatment area (311).

14. The method according to claim 12 or 13, wherein guiding the user comprises moving a roller (400) along the skin (300) and rolling the roller (400) along an axis (402) perpendicular to the movement direction (306),
wherein the roller (400) has a polygon shape having sides;
wherein each of the sides correspond to the distance (D).

15. A device movement guide (900) comprising a roller (400) adapted to be used in the light-based skin treatment device (100) according to any one of claims 4-6.
